# EUROPEAN PATENT APPLICATION

(11) **EP 3 184 042 A1**
(43) Date of publication of application: **28.06.2017**
(21) Application number: 16201003.7
(22) Date of filing: 28.11.2016
(51) Int. Cl.: A61B 5/024, A61B 5/11

(54) **ELECTRONIC DEVICE AND COMPUTER-READABLE RECORDING MEDIUM**

(30) Priority: 22.12.2015 JP 2015250584
(71) Applicant: FUJITSU LIMITED, Kawasaki-shi Kanagawa 211-8588 (JP); Murata Manufacturing Co., Ltd., Nagaokakyo-shi, Kyoto 617-8555 (JP)
(72) Inventor: KASAMA, Kouichirou, Kawasaki-shi, Kanagawa 211-8588 (JP); KOCHI, Kazutaka, Kawasaki-shi, Kanagawa 211-8588 (JP); ISHIKAWA, Hiroki, Nagaokakyo-shi, Kyoto 617-8555 (JP); OKUDA, Noriaki, Nagaokakyo-shi, Kyoto 617-8555 (JP)
(74) Representative: Hutchison, James

(57) **Abstract**

An electronic device includes: a storage that stores therein pulse rate ranges using a resting heart rate of a user as a reference to be associated with exercise amounts, respectively; an acquisition unit configured to acquire a first sensor value and a second sensor value, the first sensor detecting pulses and the second sensor detecting an acceleration; a first calculation unit configured to calculate a plurality of pulse rate candidates based on the first sensor value; a second calculation unit configured to calculate an exercise amount of the user based on the second sensor value; and a decision unit configured to specify a pulse rate range stored in the storage to be associated with the exercise amount, and to decide a pulse rate candidate included in the specified pulse rate range as a pulse rate of the user.

## Description

### FIELD

The embodiments discussed herein are related to an electronic device and a computer-readable recording medium.

### BACKGROUND

In recent years, wearable terminals that can be worn on, for example, an arm of a user are becoming popular. Because being used in a state worn on the body of a user, the wearable terminals can measure, for example, the pulse rate of the user to monitor the condition of the body of the user. An example of a pulse sensor that detects pulses of a user is a sensor that measures a reflection amount of light.

Specifically, the pulse sensor includes an LED (Light Emitting Diode) that emits light and a PD (Photo Detector) that detects light, and detects a reflection amount of light emitted from the LED toward a human body with the PD. Because the reflection amount of light changes depending on a blood flow rate, the time waveform of a light level detected with the PD changes according to pulses. Therefore, a method of subjecting the time waveform of the light level detected with the PD to, for example, FFT (Fast Fourier Transform) processing to calculate pulse rates corresponding to peaks of the resultant frequency spectrum is adopted, for example.

However, because a wearable terminal is used in a state worn on the body of a user, a sensor value of the pulse sensor includes various noises caused by body motion of the user, for example. That is, for example, in the pulse sensor that uses light reflection described above, when the FFT processing of the time waveform of a light level detected by the PD is performed, the resultant frequency spectrum may include peaks corresponding to noises. Accordingly, it is desirable to overcome difficulty in accurately measuring a pulse rate of the user.

Furthermore, to eliminate the peaks corresponding to the noises from peaks included in the frequency spectrum to specify peaks corresponding to pulses of a user, complicated processing may be performed. As a result, processing load or power consumption of a processor incorporated in the wearable terminal is increased.

Accordingly, it is desirable to provide an electronic device and a computer-readable recording medium that can calculate a pulse rate accurately by simple processing.

### SUMMARY

The present invention is defined by the appended independent claims, to which reference should now be made. Specific embodiments are defined in the dependent claims.

According to an embodiment of one aspect of the invention, an electronic device includes: a storage that stores therein pulse rate ranges using a resting heart rate of a user as a reference to be associated with exercise amounts, respectively; an acquisition unit configured to acquire a first sensor value and a second sensor value from a first sensor and a second sensor that are sensors worn on a body of the user, respectively, the first sensor detecting pulses and the second sensor detecting an acceleration; a first calculation unit configured to calculate a plurality of pulse rate candidates based on the first sensor value acquired by the acquisition unit; a second calculation unit configured to calculate an exercise amount of the user based on the second sensor value acquired by the acquisition unit; and a decision unit configured to specify a pulse rate range stored in the storage to be associated with the exercise amount calculated by the second calculation unit, and to decide a pulse rate candidate included in the specified pulse rate range among the pulse rate candidates calculated by the first calculation unit as a pulse rate of the user. The storage may be a storage unit.

### BRIEF DESCRIPTION OF DRAWINGS

Embodiments of the invention are set out, by way of example only, with reference to the following drawings, in which:
FIG. 1 is a block diagram illustrating a configuration of a portable terminal device according to a first embodiment;
FIG. 2 is a block diagram illustrating functions of a processor according to the first embodiment;
FIG. 3 is an explanatory diagram of a pulse rate candidate;
FIG. 4 is a diagram illustrating a specific example of a pulse rate candidate;
FIG. 5 is a diagram illustrating a specific example of a pulse-rate range table according to the first embodiment;
FIG. 6 is a flowchart illustrating a pulse-rate calculation process according to the first embodiment;
FIGS. 7A, 7B and 7C are explanatory diagrams of refinement of a pulse rate candidate;
FIG. 8 is a flowchart illustrating an RHR setting process;
FIG. 9 is a flowchart illustrating a pulse-rate-candidate calculation process;
FIG. 10 is a flowchart illustrating an exercise-intensity calculation process;
FIG. 11 is a block diagram illustrating functions of a processor according to a second embodiment;
FIG. 12 is a diagram illustrating a specific example of a pulse-rate range table according to the second embodiment;
FIG. 13 is a flowchart illustrating a pulse-rate calculation process according to the second embodiment; and
FIG. 14 is a flowchart illustrating a step-number calculation process.

### DESCRIPTION OF EMBODIMENTS

Preferred embodiments of the present invention will be explained with reference to accompanying drawings. The present invention is not limited to the embodiments. In the following descriptions, an as example of the electronic device, a portable terminal device that is used in a state worn on the body of a user is described; however, the techniques disclosed in the respective embodiments can be widely applicable to other types of electronic devices.

### [a] First Embodiment

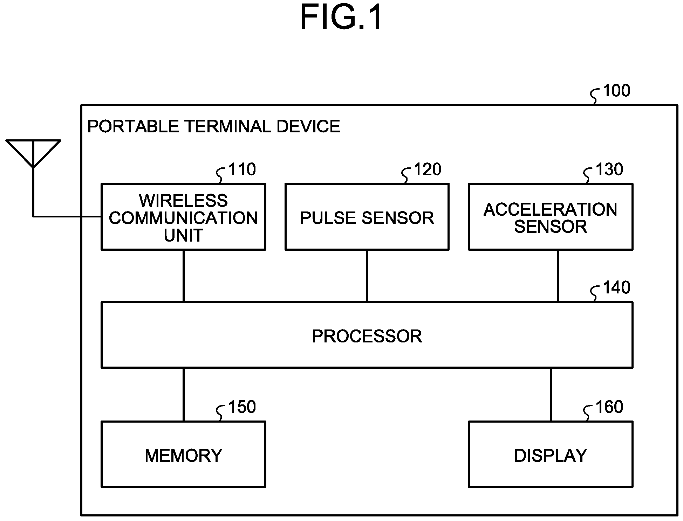

FIG. 1 is a block diagram illustrating a configuration of a portable terminal device 100 according to a first embodiment. The portable terminal device 100 illustrated in FIG. 1 has a wireless communication unit 110, a pulse sensor 120, an acceleration sensor 130, a processor 140, a memory 150, and a display 160.

The wireless communication unit 110 is, for example, a communication module including a wireless communication function using Bluetooth® and provides wireless communication with other communication terminal devices such as a smartphone. Specifically, the wireless communication unit 110 notifies other communication terminal devices of, for example, a pulse rate of a user wearing the portable terminal device 100 using BLE (Bluetooth Low Energy).

The pulse sensor 120 detects pulses of the user wearing the portable terminal device 100. Specifically, the pulse sensor 120 includes an LED and a PD, and detects reflection of light emitted from the LED with the PD to output an obtained reflection amount of light to the processor 140 as a sensor value. The light emitted toward the body of the user reflects on the user's body and an amount of light reflected at that time changes according to a flow rate of blood in the user's body. Therefore, the pulses of the user can be detected based on the sensor value of the pulse sensor 120.

The acceleration sensor 130 includes, for example, a three-axis acceleration sensor and detects respective accelerations in three axis directions of the portable terminal device 100. The acceleration sensor 130 outputs the accelerations detected by the three-axis acceleration sensor to the processor 140 as a sensor value.

The processor 140 includes, for example, a CPU (Central Processing Unit), an FPGA (Field Programmable Gate Array), or a DSP (Digital Signal Processor) and controls the entire portable terminal device 100 in an integrated manner. That is, the processor 140 performs various types of processing using the memory 150. Specifically, the processor 140 drives the pulse sensor 120 and the acceleration sensor 130 and decides the pulse rate of the user based on the pulse sensor value obtained from the pulse sensor 120 and the acceleration sensor value obtained from the acceleration sensor 130. The processor 140 performs a notification process of notifying the user of the decided pulse rate. Functions of the processor 140 will be explained in detail later.

The memory 150 includes, for example, a RAM (Random Access Memory), a ROM (Read Only Memory), or a NAND flash memory and stores therein various types of information during the processing performed by the processor 140.

The display 160 includes, for example, a liquid crystal panel and displays notification information output from the processor 140. The display 160 can be placed with a touch panel that detects contacts superposed thereon.

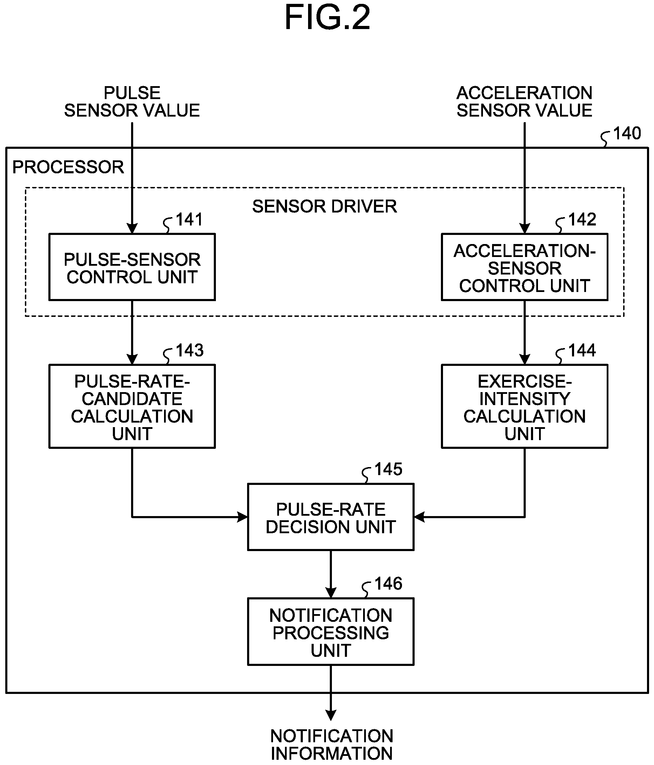

FIG. 2 is a block diagram illustrating functions of the processor 140 according to the first embodiment. The processor 140 illustrated in FIG. 2 has a pulse-sensor control unit 141, an acceleration-sensor control unit 142, a pulse-rate-candidate calculation unit 143, an exercise-intensity calculation unit 144, a pulse-rate decision unit 145, and a notification processing unit 146.

The pulse-sensor control unit 141 drives the pulse sensor 120 and acquires a reflection amount of light detected by the pulse sensor 120 with a predetermined period as a pulse sensor value. The pulse-sensor control unit 141 outputs the acquired pulse sensor value to the pulse-rate-candidate calculation unit 143.

The acceleration-sensor control unit 142 drives the acceleration sensor 130 and acquires an acceleration detected by the acceleration sensor 130 with a predetermined period as an acceleration sensor value. The acceleration-sensor control unit 142 outputs the acquired acceleration sensor value to the exercise-intensity calculation unit 144.

The pulse-sensor control unit 141 and the acceleration-sensor control unit 142 are included in a sensor driver that controls sensors included in the portable terminal device 100.

The pulse-rate-candidate calculation unit 143 filters the waveform of the pulse sensor value with a band-pass filter that passes a predetermined frequency band to eliminate noises outside the band. The pulse-rate-candidate calculation unit 143 performs FFT processing of the waveform after noise elimination to obtain a frequency spectrum of the waveform of the pulse sensor value. The pulse-rate-candidate calculation unit 143 detects peaks in the frequency spectrum and determines the number of beats having a frequency (hereinafter, "peak frequency") corresponding to each of the peaks per unit time (one minute, for example) as a pulse rate candidate.

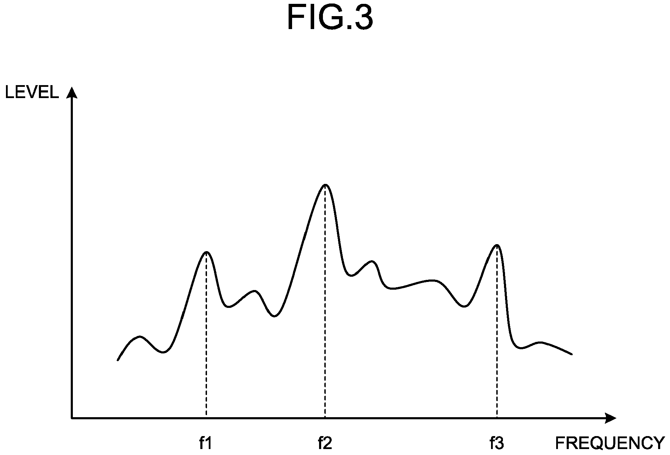

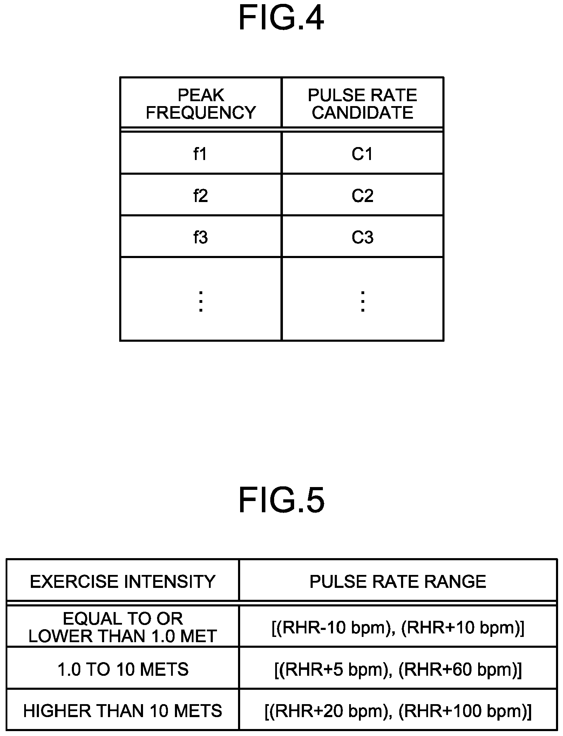

To cite a specific example, the pulse-rate-candidate calculation unit 143 performs the FFT processing of the waveform of the pulse sensor value and obtains a frequency spectrum as illustrated in FIG. 3, for example. This frequency spectrum has three peak frequencies f1, f2, and f3. Accordingly, the pulse-rate-candidate calculation unit 143 detects the three peak frequencies f1, f2, and f3 from the frequency spectrum. Because the numbers of beats corresponding to these peak frequencies f1, f2, and f3 are pulse rate candidates, respectively, the pulse-rate-candidate calculation unit 143 obtains pulse rate candidates corresponding to the respective peak frequencies. In this case, for example, as illustrated in FIG. 4, a pulse rate candidate C1 corresponding to the peak frequency f1, a pulse rate candidate C2 corresponding to the peak frequency f2, and a pulse rate candidate C3 corresponding to the peak frequency f3 are obtained.

The pulse-rate-candidate calculation unit 143 reads pulse rates having been decided and stored in a past predetermined time from the memory 150 and performs refinement of the pulse rate candidates based on the read pulse rates. That is, the pulse-rate-candidate calculation unit 143 selects pulse rate candidates within a predetermined range around the past pulse rates and stores the selected pulse rate candidates in the memory 150. The pulse-rate-candidate calculation unit 143 does not need to perform the refinement of the pulse rate candidates based on the past pulse rates and may store the pulse rate candidates corresponding to all the peak frequencies in the memory 150.

The exercise-intensity calculation unit 144 calculates a current exercise intensity of the user based on the acceleration sensor value. Because the acceleration sensor 130 can detect motion of the portable terminal device 100 worn on the user's body, the acceleration sensor 130 can calculate an exercise intensity of the user from the acceleration sensor value indicating an amount of the motion. For example, METs (Metabolic Equivalents of Task) can be used for the exercise intensity. The exercise intensity is expressed with a relative value based on the exercise intensity at rest, which set at 1.0 MET.

The pulse-rate decision unit 145 sets a resting heart rate RHR of the user being in a resting state based on the exercise intensity calculated by the exercise-intensity calculation unit 144. The pulse-rate decision unit 145 then decides one of the pulse rate candidates stored in the memory 150 as the pulse rate based on the set resting heart rate RHR and the exercise intensity calculated by the exercise-intensity calculation unit 144.

Specifically, the pulse-rate decision unit 145 detects that the user is in a resting state, for example, when a state where the exercise intensity is equal to or lower than a predetermined value has continued for a predetermined time. The pulse-rate decision unit 145 sets a pulse rate candidate calculated by the pulse-rate-candidate calculation unit 143 in a case where a resting state is detected as the resting heart rate RHR. When the user is in the resting state, there is almost no body motion of the user and thus a few noises are included in the pulse sensor value. Accordingly, it is considered that one pulse rate candidate is calculated by the pulse-rate-candidate calculation unit 143 in the case where a resting state of the user is detected. Therefore, the calculated pulse rate candidate is set as the resting heart rate RHR.

After the resting heart rate RHR of the user is set, the pulse-rate decision unit 145 decides a pulse rate candidate included in a range using the resting heart rate RHR as a reference to be the pulse rate of the user. That is, the pulse-rate decision unit 145 specifies a pulse rate range corresponding to the exercise intensity calculated by the exercise-intensity calculation unit 144. At that time, for example, the pulse-rate decision unit 145 reads a pulse-rate range table as illustrated in FIG. 5 from the memory 150 and refers to the pulse-rate range table to specify the pulse rate range corresponding to the exercise intensity.

The pulse-rate range table illustrated in FIG. 5 represents that, when the exercise intensity is equal to or lower than 1.0 MET, the minimum value of the corresponding pulse rate range is the resting heart rate RHR-10 bpm (beats per minute) and the maximum value thereof is the resting heart rate RHR+10 bpm. That is, when the exercise intensity is equal to or lower than 1.0 MET, a pulse rate range that is plus or minus 10 bpm of the resting heart rate RHR is specified. Similarly, when the exercise intensity is between 1.0 MET and 10 METs, a pulse rate range that is 5 to 60 bpm higher than the resting heart rate RHR is specified. When the exercise intensity exceeds 10 METs, a pulse rate range that is 20 to 100 bpm higher than the resting heart rate RHR is specified. In this way, in the pulse-rate range table, a higher pulse rate range using the resting heart rate RHR as a reference is associated with a higher exercise intensity.

When having specified the pulse rate range corresponding to the exercise intensity until the current time with reference to the pulse-rate range table in the manner described above, the pulse-rate decision unit 145 decides a pulse rate candidate included in the specified pulse rate range among the pulse rate candidates stored in the memory 150 as the pulse rate of the user. At that time, when there are plural pulse rate candidates included in the specified pulse rate range, a pulse rate range in which the level of the corresponding peak is highest among the pulse rate candidates is decided as the pulse rate of the user.

Upon decision of the pulse rate of the user by the pulse-rate decision unit 145, the notification processing unit 146 generates notification information for notifying of the decided pulse rate and causes the display 160 to display the generated notification information. The notification processing unit 146 may cause the wireless communication unit 110 to transmit the generated notification information therefrom.

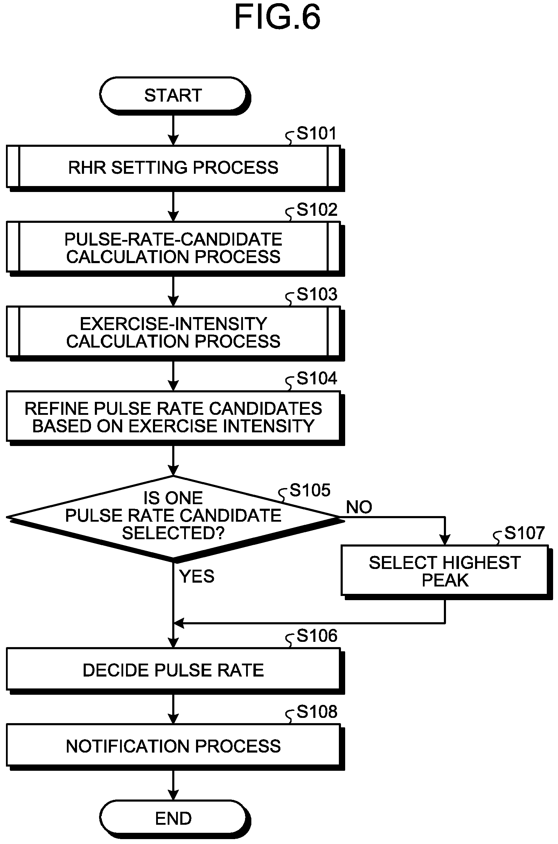

A pulse-rate calculation process performed by the portable terminal device 100 configured as described above is explained next with reference to a flowchart illustrated in FIG. 6.

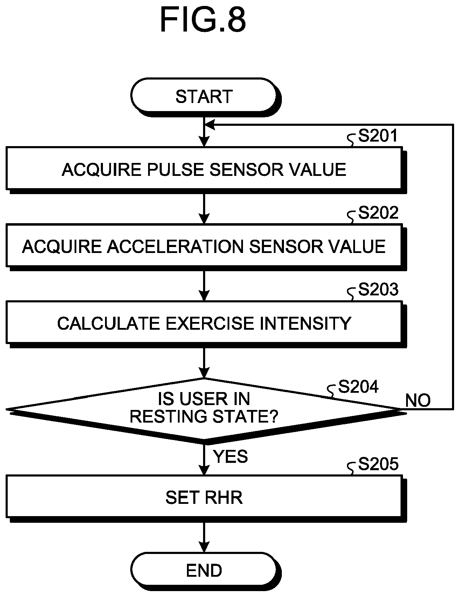

When the portable terminal device 100 is worn on the body of a user, the resting heart rate RHR of the user is set as an advance preparation for pulse rate calculation (Step S101). That is, when the exercise intensity calculated by the exercise-intensity calculation unit 144 meets a predetermined condition, the user is determined to be in a resting state and a pulse rate candidate calculated in the resting state by the pulse-rate-candidate calculation unit 143 is set as the resting heart rate RHR. This RHR setting process is explained in detail later with reference to FIG. 8.

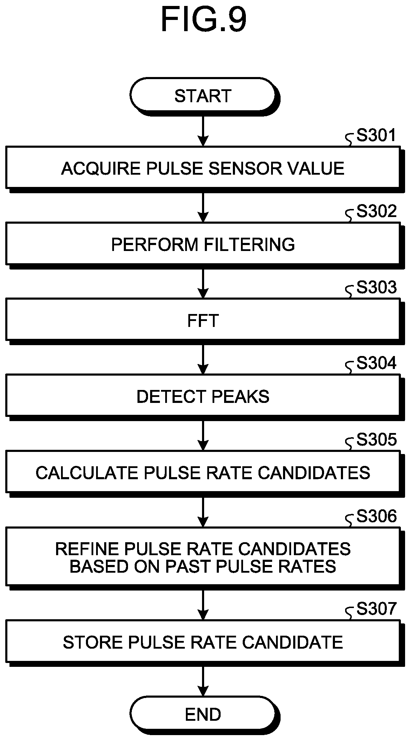

Upon setting of the resting heart rate RHR, the pulse-rate-candidate calculation unit 143 calculates a plurality of pulse rate candidates (Step S102). That is, the pulse-rate-candidate calculation unit 143 performs the FFT processing of the waveform of the pulse sensor value and calculates pulse rate candidates corresponding to plural peak frequencies of a resultant frequency spectrum, respectively. This pulse-rate-candidate calculation process is explained in detail later with reference to FIG. 9.

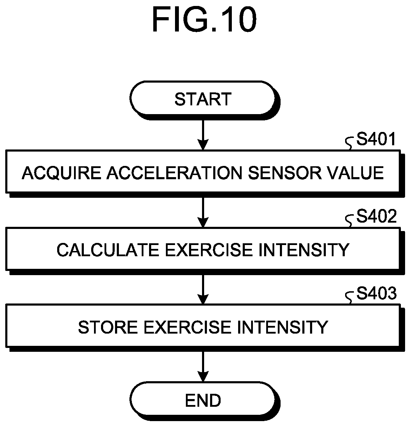

At the same time as calculation of the pulse rate candidates, the exercise-intensity calculation unit 144 calculates an exercise intensity based on the acceleration sensor value (Step S103). That is, at the same time as the pulse-rate-candidate calculation unit 143 calculates plural pulse rate candidates, the exercise-intensity calculation unit 144 calculates an exercise intensity of the user during this period. This exercise-intensity calculation process is explained in detail later with reference to FIG. 10.

Subsequently, the pulse-rate decision unit 145 performs refinement of the pulse rate candidates based on the exercise intensity (Step S104). Specifically, the pulse-rate decision unit 145 specifies a pulse rate range corresponding to the exercise intensity in a predetermined period until the current time with reference to the pulse-rate range table stored in the memory 150. As described above, in the pulse-rate range table, a higher pulse rate range using the resting heart rate RHR as a reference is associated with a higher extension intensity. Accordingly, when the exercise intensity is high, the pulse-rate decision unit 145 specifies a pulse rate range including high pulse rates using the resting heart rate RHR as a reference.

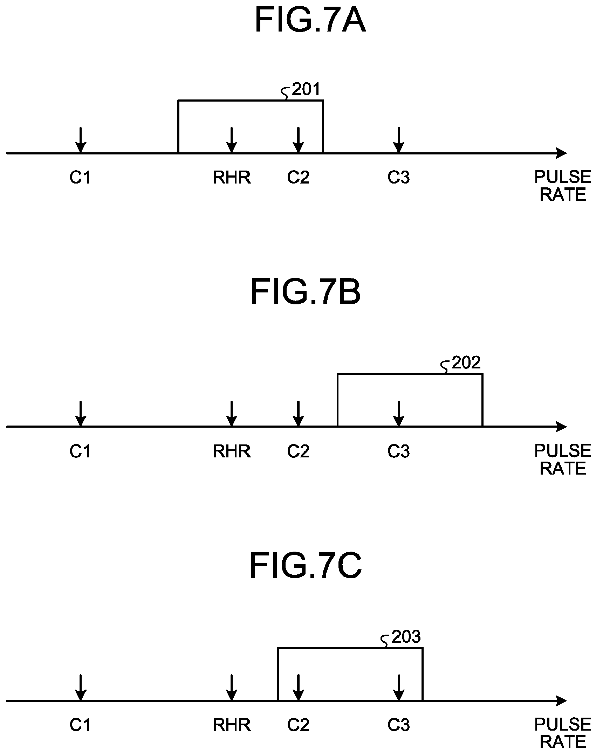

When the pulse rate range is specified, the pulse-rate decision unit 145 selects a pulse rate candidate included in the specified pulse rate range among the pulse rate candidates. Specific examples are cited. For example, when a pulse rate range 201 using the resting heart rate RHR as a reference is specified based on the exercise intensity, a pulse rate candidate C2 is selected from among pulse rate candidates C1, C2, and C3 as illustrated in FIG. 7A. Similarly, for example, when a pulse rate range 202 using the resting heart rate RHR as a reference is specified based on the exercise intensity, the pulse rate candidate C3 is selected from among the pulse rate candidates C1, C2, and C3 as illustrated in FIG. 7B. For example, when a pulse rate range 203 using the resting heart rate RHR as a reference is specified based on the exercise intensity, the pulse rate candidates C2 and C3 are selected from among the pulse rate candidates C1, C2, and C3 as illustrated in FIG. 7C.

Referring back to FIG. 6, upon selection of the pulse rate candidate or the pulse rate candidates included in the specified pulse rate range, whether one pulse rate candidate is selected is determined (Step S105). When the determination result indicates that one pulse rate candidate is selected (YES at Step S105), this pulse rate candidate is decided as the pulse rate of the user (Step S106). That is, in the examples described above, when the number of pulse rate candidates within the specific pulse rate range is one as illustrated in FIGS. 7A and 7B, the pulse rate candidate C2 or C3 is decided as the pulse rate of the user.

On the other hand, when plural pulse rate candidates are selected (NO at Step S105), a pulse rate candidate having a highest level of the corresponding peak in the frequency spectrum is selected from among the selected pulse rate candidates (Step S107). The selected pulse rate candidate corresponding to the highest peak is decided as the pulse rate of the user (Step S106). That is, in the examples described above, when plural pulse rate candidates are included in the specified pulse rate range as illustrated in FIG. 7C, a pulse rate candidate corresponding to a highest peak out of the pulse rate candidates C2 and C3 is decided as the pulse rate of the user.

When the pulse rate of the user is decided by the pulse-rate decision unit 145 in this way, the notification processing unit 146 generates notification information to notify of the pulse rate. The notification processing unit 146 performs a notification process of causing the display 160 to display the notification information or causing the wireless communication unit 110 to transmit the notification information therefrom (Step S108).

As described above, when plural peaks are included in the frequency spectrum obtained by the FFT processing of the waveform of the pulse sensor value, a pulse rate candidate within a pulse rate range based on the exercise intensity and the resting heart rate RHR is selected from pulse rate candidates corresponding to respective peak frequencies to decide the pulse rate of the user. Accordingly, the pulse rate can be calculated accurately by simple processing while influences of noises, for example, caused by body motion of the user are eliminated.

The process at each of Steps S101 to S103 in the pulse-rate calculation process described above is explained next. FIG. 8 is a flowchart illustrating the RHR setting process at Step S101 described above.

The RHR setting process is performed as an advance preparation for pulse rate calculation when the portable terminal device 100 is worn on the body of a user. Specifically, the pulse-sensor control unit 141 acquires the pulse sensor value from the pulse sensor 120 (Step S201) and similarly the acceleration-sensor control unit 142 acquires the acceleration sensor value from the acceleration sensor 130 (Step S202).

Upon acquisition of the pulse sensor value, the pulse-rate-candidate calculation unit 143 performs filtering and FFT processing of the waveform of the pulse sensor value to detect peaks in the frequency spectrum. Meanwhile, upon acquisition of the acceleration sensor value, the exercise-intensity calculation unit 144 calculates an exercise intensity (Step S203). The pulse-rate decision unit 145 is notified of the calculated exercise intensity and the pulse-rate decision unit 145 determines whether the user is in a resting state based on the exercise intensity (Step S204).

That is, the pulse-rate decision unit 145 determines whether the exercise intensity meets a predetermined condition. The predetermined condition is a condition enabling to determine that there is no factor increasing the pulse rate of the user, such as continuation of a period in which the exercise intensity is 1.0 MET for three minutes or longer. While the determination result is indicating that the exercise intensity does not meet the predetermined condition and that the user is not in a resting state (NO at Step S204), acquisition of the pulse sensor value and the acceleration sensor value is repeated to wait for a resting state of the user.

When it is determined that the exercise intensity meets the predetermined condition and that the user in a resting state (YES at Step S204), a pulse rate corresponding to a peak detected at that time by the pulse-rate-candidate calculation unit 143 is decided as the resting heart rate RHR. When the user in a resting state, the body motion of the user is fairly small and the waveform of the pulse sensor value includes few noises. Therefore, only one peak corresponding to the pulse rate of the user is detected from the frequency spectrum in many cases when the user in a resting state and thus a peak frequency corresponding to the resting heart rate RHR can be decided easily. Even when plural peaks are included in the frequency spectrum, the levels of peaks corresponding to noises are considerably smaller than the levels of the peaks corresponding to pulses because the user is in a resting state, and the peak frequency corresponding to the resting heart rate RHR can be decided easily.

When the pulse rate corresponding to a peak in the frequency spectrum of a case where the user is in a resting state is decided, the pulse-rate decision unit 145 sets the decided pulse rate as the resting heart rate RHR (Step S205). That is, the pulse-rate decision unit 145 obtains the resting heart rate RHR unique to the user and sets pulse rate ranges using the resting heart rate RHR as a reference in the pulse-rate range table. Therefore, the pulse rate ranges according to the resting heart rate RHR of the user are set in the pulse-rate range table illustrated in FIG. 5, for example, and the pulse-rate range table unique to the user is stored in the memory 150.

FIG. 9 is a flowchart illustrating the pulse-rate-candidate calculation process at Step S102 described above. The pulse-rate-candidate calculation process is performed mainly by the pulse-rate-candidate calculation unit 143.

First, the pulse-sensor control unit 141 acquires the pulse sensor value from the pulse sensor 120 (Step S301). The waveform of the acquired pulse sensor value is subjected by the pulse-rate-candidate calculation unit 143 to filtering that transmits a predetermined band (Step S302). The filtering is for eliminating noises of a frequency band that is improbable for the pulse rate and is performed using a band-pass filter. In this way, quite low frequency components and quite high frequency components are eliminated from the waveform of the pulse sensor value.

The waveform of the pulse sensor value having passed through the band-pass filter is subjected to the FFT processing (Step S303). That is, a time waveform of the pulse sensor value is converted into a frequency spectrum of a frequency domain. Subsequently, the pulse-rate-candidate calculation unit 143 detects peaks of the frequency spectrum (Step S304). Peaks in the frequency spectrum indicate beats included in the waveform of the pulse sensor value and thus the number of beats having a peak frequency per unit time is calculated as a pulse rate candidate (Step S305). Because the user is not always in a resting state at that time, many noises occur, for example, due to body motion of the user and a plurality of peaks are detected from the frequency spectrum. As a result, a plurality of pulse rate candidates are calculated.

When the pulse rate candidates are calculated, refinement of the pulse rate candidates is performed based on a history of pulse rates having been decided by the pulse-rate decision unit 145 in the past (Step S306). Specifically, a pulse rate candidate within a predetermined range is selected and other pulse rate candidates are discarded based on the history of the past pulse rates. The selected and left pulse rate candidate is stored in the memory 150 (Step S307).

The refinement of the pulse rate candidates based on the history of the past pulse rates at Step S306 described above does not always need to be performed. Even when the refinement of the pulse rate candidates based on the history of the past pulse rates is performed, plural pulse rate candidates can be left and stored in the memory 150. Any one of the pulse rate candidates thus stored in the memory 150 is the pulse rate of the user.

FIG. 10 is a flowchart illustrating the exercise-intensity calculation process at Step S103 described above. The exercise-intensity calculation process is performed mainly by the exercise-intensity calculation unit 144.

First, the acceleration-sensor control unit 142 acquires the acceleration sensor value from the acceleration sensor 130 (Step S401). The acquired acceleration sensor value is a measurement value of an acceleration occurring with motion of the user. Therefore, an amount of exercise of the user can be estimated from the acceleration sensor value and thus an exercise intensity of the user is calculated by the exercise-intensity calculation unit 144 based on the acceleration sensor value (Step S402). The calculated exercise intensity is temporarily stored in the memory 150 (Step S403).

Generally, when a user performs exercise with a high exercise intensity, the pulse rate of the user increases. Accordingly, an estimated pulse rate range of the user differs according to the exercise intensity. Therefore, for example, in the pulse-rate range table illustrated in FIG. 5, pulse rate ranges are associated with exercise intensities, respectively, and a pulse rate range according to an exercise intensity is specified when the pulse rate of the user is to be decided from plural pulse rate candidates.

As described above, according to the present embodiment, a resting state of a user is detected based on an exercise intensity of the user calculated from the acceleration sensor value and pulse rate ranges for respective exercise intensities using a resting heart rate of the user in a resting state as a reference are stored in advance. The waveform of the pulse sensor value is then subjected to the FFT processing and a pulse rate candidate included in a pulse rate range according to an exercise intensity among pulse rate candidates corresponding to peaks of the resultant frequency spectrum is decided as the pulse rate of the user. Accordingly, the pulse rate can be calculated accurately by simple processing while influences of noises caused by body motion of the user, for example, are eliminated.

### [b] Second Embodiment

A second embodiment is characterized in that pulse rate candidates are refined using the number of steps as well as the exercise intensity.

The configuration of a portable terminal device according to the second embodiment is identical to that of the portable terminal device 100 (FIG. 1) according to the first embodiment, and thus explanations thereof are omitted. In the second embodiment, functions of the processor 140 are different from those in the first embodiment.

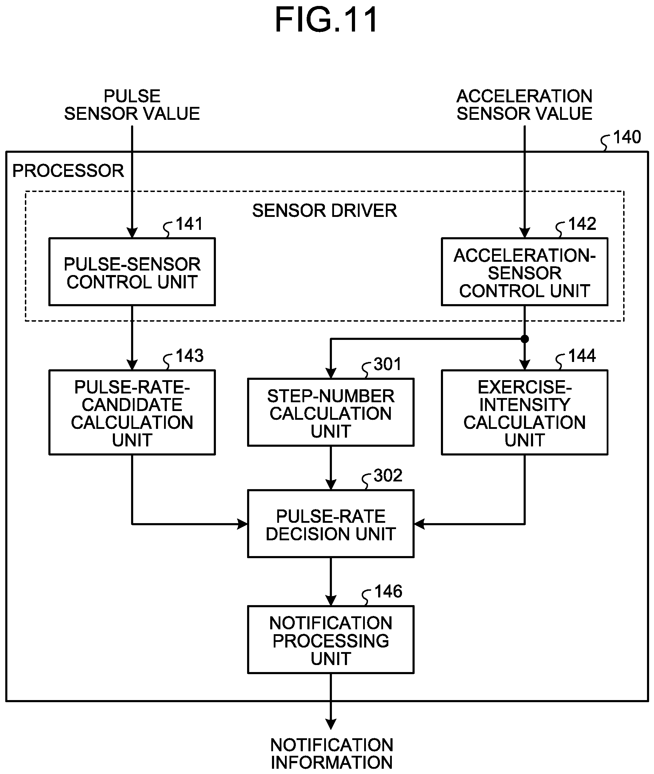

FIG. 11 is a block diagram illustrating functions of the processor 140 according to the second embodiment. In FIG. 11, like parts in FIG. 2 are denoted by like reference signs to omit explanations thereof. The processor 140 illustrated in FIG. 11 adopts a configuration in which a step-number calculation unit 301 is additionally provided to the processor 140 illustrated in FIG. 2 and the pulse-rate decision unit 145 is replaced with a pulse-rate decision unit 302.

The step-number calculation unit 301 calculates the number of steps of the user based on an acceleration sensor value. Specifically, the step-number calculation unit 301 estimates a direction of a gravitational acceleration based on an acceleration sensor value of a three-axis acceleration sensor, for example, and extracts a change pattern of an acceleration due to walking from a change pattern of an acceleration in a direction parallel to the gravitational acceleration, for example. The step-number calculation unit 301 calculates the number of steps of the user based on the extracted change pattern of the acceleration due to walking.

The pulse-rate decision unit 302 sets a resting heart rate RHR of the user in a resting state based on the exercise intensity calculated by the exercise-intensity calculation unit 144 and the number of steps calculated by the step-number calculation unit 301. The pulse-rate decision unit 302 then decides one of the pulse rate candidates stored in the memory 150 based on the set resting heart rate RHR, the exercise intensity, and the number of steps as the pulse rate.

Specifically, for example, when a state in which the exercise intensity or the number of steps per predetermined time is equal to or lower than a predetermined value has continued for a certain time, the pulse-rate decision unit 302 detects a resting state of the user. The pulse-rate decision unit 302 then sets a pulse rate candidate calculated by the pulse-rate-candidate calculation unit 143 at the time when the resting state is detected as the resting heart rate RHR.

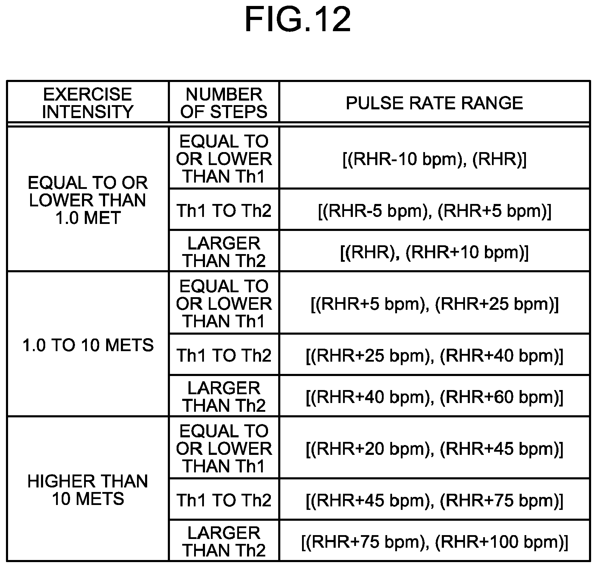

After the resting heart rate RHR of the user is set, the pulse-rate decision unit 302 decides a pulse rate candidate in a range using the resting heart rate RHR as a reference as the pulse rate of the user. That is, the pulse-rate decision unit 302 specifies a pulse rate range corresponding to the exercise intensity calculated by the exercise-intensity calculation unit 144 and the number of steps calculated by the step-number calculation unit 301. At that time, the pulse-rate decision unit 302 reads, for example, a pulse-rate range table as illustrated in FIG. 12 from the memory 150 and specifies a pulse rate range corresponding to the exercise intensity and the number of steps per predetermined time with reference to the pulse-rate range table.

In the pulse-rate range table illustrated in FIG. 12, three levels of exercise intensities are subdivided into three levels according to the numbers of steps per predetermined time, respectively. That is, the exercise intensity is divided into three levels "equal to or lower than 1.0 MET", "1.0 MET to 10 METs", and "higher than 10 METs". Each of the levels of exercise intensities is subdivided according to the numbers of steps per minute to be bounded at a threshold Th1 and a threshold Th2, respectively. The threshold Th2 is a value larger than the threshold Th1.

The table represents that, for example, when the exercise intensity is equal to or lower than 1.0 MET and the number of steps is equal to or lower than the threshold Th1, the minimum value of the corresponding pulse rate range is the resting heart rate RHR-10 bpm and the maximum value thereof is the resting heart rate RHR. That is, when the exercise intensity is equal to or lower than 1.0 MET and the number of steps is equal to or lower than the threshold Th1, a pulse rate range that is 0 to 10 bpm lower than the resting heart rate RHR is specified. The table also represents that, when the number of steps is between the threshold Th1 and the threshold Th2 while the exercise intensity is equal to or lower than 1.0 MET, the minimum value of the corresponding pulse rate range is the resting heart rate RHR-5 bpm and the maximum value thereof is the resting heart rate RHR+5 bpm. That is, when the exercise intensity is equal to or lower than 1.0 MET and the number of steps is between the threshold Th1 and the threshold Th2, a pulse rate range that is plus or minus 5 bpm of the resting heart rate RHR is specified. As described above, in the pulse-rate range table, a higher pulse rate range using the resting heart rate RHR as a reference is associated with a larger number of steps even when the exercise intensity is the same.

When the exercise intensity is between 1.0 MET and 10 METs and the number of steps is equal to or lower than the threshold Th1, a pulse rate range that is 5 to 25 bpm higher than the resting heart rate RHR is specified. When the exercise intensity is higher than 10 METs and the number of steps is equal to or lower than the threshold Th1, a pulse rate range that is 20 to 45 bpm higher than the resting heart rate RHR is specified. As described above, in the pulse-rate range table, a higher pulse rate range using the resting heart rate RHR as a reference is associated with a higher exercise intensity.

In the pulse-rate range table according to the present embodiment, the pulse rate ranges are subdivided using the number of steps per predetermined time in addition to the exercise intensity, which can narrow the pulse rate ranges corresponding to the individual exercise intensities and numbers of steps and can refine the pulse rate candidates more reliably.

Upon specification of a pulse rate range corresponding to the exercise intensity and the number of steps until the current time with reference to the pulse-rate range table in the manner described above, the pulse-rate decision unit 302 decides a pulse rate candidate included in the specified pulse rate range among the pulse rate candidates stored in the memory 150 as the pulse rate of the user. At that time, when plural pulse rate candidates are included in the specified pulse rate range, a pulse rate candidate having a highest level of the corresponding peak among these pulse rate candidates is decided as the pulse rate of the user.

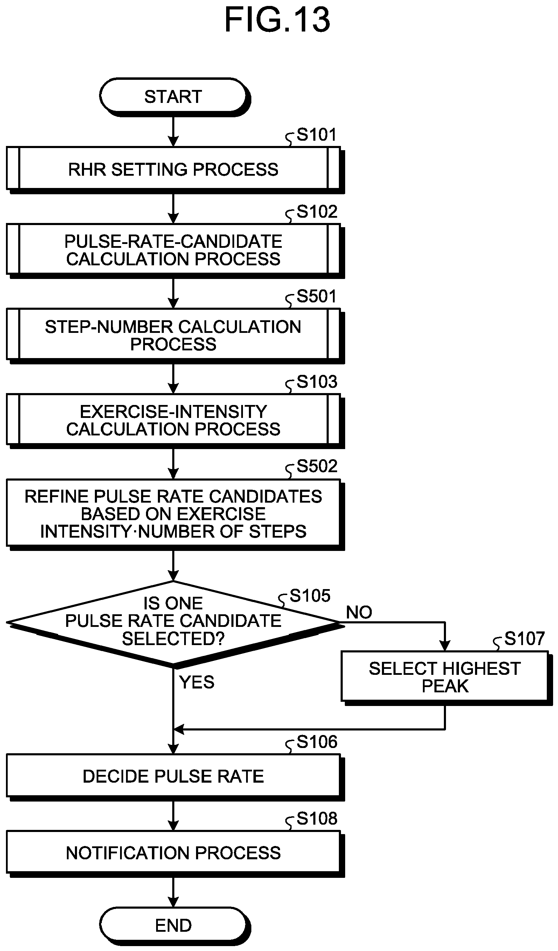

A pulse-rate calculation process performed by the portable terminal device 100 configured as described above is explained next with reference to a flowchart illustrated in FIG. 13. In FIG. 13, like parts in FIG. 6 are denoted by like reference signs to omit detailed explanations thereof.

When the portable terminal device 100 is worn on the body of a user, the resting heart rate RHR of the user is set as an advance preparation for pulse rate calculation (Step S101). At the time of setting of the resting heart rate RHR, whether the user is in a resting state can be determined based on the exercise intensity similarly in the first embodiment. Whether the user is in a resting state can alternatively be determined based on the number of steps. That is, when the number of steps calculated by the step-number calculation unit 301 meets a predetermined condition, the user can be determined to be in a resting state. Upon setting of the resting heart rate RHR, the pulse-rate-candidate calculation unit 143 calculates a plurality of pulse rate candidates (Step S102).

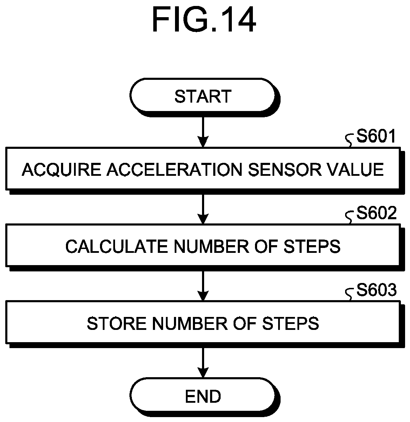

At the same time as the calculation of the pulse rate candidates, the step-number calculation unit 301 calculates the number of steps based on the acceleration sensor value (Step S501). That is, at the same time as the pulse-rate-candidate calculation unit 143 calculates the pulse rate candidates, the step-number calculation unit 301 extracts a pattern corresponding to walking from time-series changes of the acceleration sensor value and calculates the number of steps. This step-number calculation process is explained in detail later with reference to FIG. 14.

At the same time as the calculation of the pulse rate candidates and the number of steps, the exercise-intensity calculation unit 144 calculates an exercise intensity based on the acceleration sensor value (Step S103). Subsequently, the pulse-rate decision unit 302 performs refinement of the pulse rate candidates based on the exercise intensity and the number of steps (Step S502). Specifically, the pulse-rate decision unit 302 refers to the pulse-rate range table stored in the memory 150 to specify a pulse rate range corresponding to the exercise intensity and the number of steps in a predetermined period until the current time. As described above, in the pulse-rate range table, a range of higher pulse rates using the resting heart rate RHR as a reference is associated with a higher exercise intensity. Therefore, when the exercise intensity is high and the number of steps is large, the pulse-rate decision unit 302 specifies a pulse rate range including higher pulse rates using the resting heart rate RHR as a reference.

Upon specification of the pulse rate range, the pulse-rate decision unit 302 selects pulse rate candidates included in the specified pulse rate range from among the plural pulse rate candidates. Subsequently, whether one pulse rate candidate is selected is determined (Step S105). When one pulse rate candidate is selected (YES at Step S105), this pulse rate candidate is decided as the pulse rate of the user (Step S106). When plural pulse rate candidates are selected (NO at Step S105), a pulse rate candidate having a highest level of the corresponding peak in the frequency spectrum is selected from among the selected pulse rate candidates (Step S107). The selected pulse rate candidate corresponding to the highest peak is decided as the pulse rate of the user (Step S106).

When the pulse rate of the user is decided by the pulse-rate decision unit 302 in this way, the notification processing unit 146 generates notification information for notifying of the pulse rate. The notification processing unit 146 performs a notification process of causing the display 160 to display the notification information or causing the wireless communication unit 110 to transmit the notification information (Step S108).

As described above, when the frequency spectrum obtained by the FFT processing of the waveform of the pulse sensor value includes a plurality of peaks, a pulse rate candidate within a pulse rate range based on the exercise intensity, the number of steps, and the resting heart rate RHR is selected from pulse rate candidates corresponding to respective peak frequencies to decide the pulse rate of the user. Accordingly, the pulse rate can be calculated accurately by simple processing while influences of noises caused by, for example, body motion of the user are eliminated. Furthermore, because the pulse rate range is specified using also the number of steps as well as the exercise intensity, a narrower pulse rate range can be specified and the pulse rate candidates can be refined reliably.

FIG. 14 is a flowchart illustrating a step-number calculation process at Step S501 described above. The step-number calculation process is performed mainly by the step-number calculation unit 301.

First, the acceleration-sensor control unit 142 acquires the acceleration sensor value from the acceleration sensor 130 (Step S601). Because the acquired acceleration sensor value is a measurement value of a three-axis acceleration, the direction of a gravitational acceleration can be estimated from the acceleration sensor value. Subsequently, the step-number calculation unit 301 extracts a change pattern of an acceleration due to walking from a change pattern of an acceleration in a direction parallel to the gravitational acceleration. The number of steps of the user is calculated from the extracted change pattern of the acceleration due to walking (Step S602).

The calculation of the number of steps by the step-number calculation unit 301 can be performed continuously while the user is wearing the portable terminal device on the body. That is, the number of steps of the user can always be calculated to count a cumulative number of steps regardless of the pulse-rate calculation process. The calculated number of steps is temporarily stored in the memory 150 (Step S603).

Generally, when the number of steps of the user per predetermined time increases, the pulse rate of the user increases. Accordingly, an estimated pulse rate range of the user differs according to the number of steps. Therefore, for example, in the pulse-rate range table illustrated in FIG. 12, pulse rate ranges are associated with the numbers of steps per predetermined time, respectively. When the pulse rate of the user is to be decided from plural pulse rate candidates, a pulse rate range corresponding also to the number of steps as well as the exercise intensity is specified.

As described above, according to the present embodiment, a resting state of the user is detected based on the exercise intensity or the number of steps of the user calculated from the acceleration sensor value and pulse rate ranges for respective exercise intensities and numbers of steps using a resting heart rate of the user in a resting state as a reference are stored in advance. The waveform of the pulse sensor value is then subjected to the FFT processing and a pulse rate candidate included in a pulse rate range according to the exercise intensity and the number of steps among pulse rate candidates corresponding to peaks of a resultant frequency spectrum is decided as the pulse rate of the user. Accordingly, the pulse rate can be calculated accurately by simple processing while influences of noises caused by, for example, body motion of the user are eliminated. Furthermore, because the pulse rate range is specified using also the number of steps as well as the exercise intensity, a narrower pulse rate range can be specified and the pulse rate candidates can be refined reliably.

While the step-number calculation unit 301 calculates the number of steps and the pulse rate range is specified using the number of steps in the second embodiment, the number of steps does not need to be always used when it can be determined that the pulse rate of the user changes. That is, for example, when a function such as a GPS (Global Positioning System) or a wireless LAN (Local Area Network) is used, movement of the portable terminal device can be detected and whether the user is walking can be determined based on a movement distance or a movement speed. When the user is walking, it can be determined that the pulse rate of the user changes based on the movement distance or the movement speed and accordingly a pulse rate range corresponding to the movement distance and the movement speed instead of the number of steps can be specified.

In the above embodiments, the pulse rate of a user is calculated by the portable terminal device worn on the body of the user. However, the pulse-rate calculation process can be performed by other information processors. That is, a portable terminal device worn on the body of a user can transmit the sensor values obtained by the pulse sensor 120 and the acceleration sensor 130 from the wireless communication unit 110 to other information processors and the information processors having received the sensor values can perform the pulse-rate calculation process. It is alternatively possible that the sensor values are transferred, for example, to a predetermined server via the Internet and the pulse-rate calculation process is performed in the predetermined server.

The pulse-rate calculation process described in the above embodiments can be described as a computer-executable program. In this case, this program can be stored in a computer-readable recording medium and loaded into a computer. Examples of the computer-readable recording medium are transportable recoding media such as a CD-ROM, a DVD disk, a USB memory and semiconductor memories such as a flash memory.

According to an embodiment of one aspect of the electronic device and the computer-readable recording medium disclosed in the present application, a pulse rate can be calculated accurately by simple processing.

In any of the above aspects, the various features may be implemented in hardware, or as software modules running on one or more processors. Features of one aspect may be applied to any of the other aspects.

The invention also provides a computer program or a computer program product for carrying out any of the methods described herein, and a computer readable medium having stored thereon a program for carrying out any of the methods described herein. A computer program embodying the invention may be stored on a computer-readable medium, or it could, for example, be in the form of a signal such as a downloadable data signal provided from an Internet website, or it could be in any other form.

## Claims

1. An electronic device (100) comprising:
a storage (150) that stores therein pulse rate ranges using a resting heart rate of a user as a reference to be associated with exercise amounts, respectively;
an acquisition unit (141, 142) configured to acquire a first sensor value and a second sensor value from a first sensor (120) and a second sensor (130) that are sensors worn on a body of the user, respectively, the first sensor (120) detecting pulses and the second sensor (130) detecting an acceleration;
a first calculation unit (143) configured to calculate a plurality of pulse rate candidates based on the first sensor value acquired by the acquisition unit (141);
a second calculation unit (144, 301) configured to calculate an exercise amount of the user based on the second sensor value acquired by the acquisition unit (142); and
a decision unit (145, 302) configured to specify a pulse rate range stored in the storage (150) to be associated with the exercise amount calculated by the second calculation unit (144, 301), and to decide a pulse rate candidate included in the specified pulse rate range among the pulse rate candidates calculated by the first calculation unit (143) as a pulse rate of the user.

2. The electronic device (100) according to claim 1, wherein
the storage (150) stores therein pulse rate ranges to be associated with exercise intensities, respectively, and
the second calculation unit (144) calculates an exercise intensity of the user based on the second sensor value.

3. The electronic device (100) according to claim 1 or 2, wherein
the storage (150) stores therein pulse rate ranges to be associated with exercise intensities and numbers of steps per predetermined time, respectively, and
the second calculation unit (144, 301) calculates an exercise intensity and number of steps per predetermined time of the user based on the second sensor value.

4. The electronic device (100) according to any of the preceding claims, wherein the storage (150) stores therein pulse rate ranges for which a pulse rate detected by the first sensor (120) when the exercise amount of the user calculated by the second calculation unit (144, 301) meets a predetermined condition is set as a resting heart rate of the user.

5. The electronic device (100) according to any of the preceding claims, wherein the first calculation unit (143) converts a time waveform of the first sensor value to obtain a frequency spectrum, detects a plurality of peaks in the obtained frequency spectrum, and calculates pulse rate candidates corresponding to the detected peaks, respectively.

6. The electronic device (100) according to claim 5, wherein when the specified pulse rate range includes a plurality of pulse rate candidates, the decision unit (145, 302) decides, among the pulse rate candidates, a pulse rate candidate that corresponds to a highest peak detected by the first calculation unit (143) as the pulse rate of the user.

7. A computer-readable recording medium having stored therein a program that causes a computer to execute a process comprising:
acquiring a first sensor value and a second sensor value from a first sensor (120) and a second sensor (130) that are sensors worn on a body of a user, respectively, the first sensor (120) detecting pulses and the second sensor (130) detecting an acceleration;
calculating a plurality of pulse rate candidates based on the acquired first sensor value;
calculating an exercise amount of the user based on the acquired second sensor value; and
reading a pulse rate range stored to be associated with the calculated exercise amount from a storage (150) that stores therein pulse rate ranges using a resting heart rate of the user as a reference to be associated with exercise amounts, respectively, and deciding a pulse rate candidate included in the read pulse rate range among the calculated pulse rate candidates as a pulse rate of the user.
